# EUROPEAN PATENT SPECIFICATION

(11) **EP 1 905 427 B1**
(45) Date of publication and mention of the grant of the patent: **24.11.2010**
(21) Application number: 06121457.3
(22) Date of filing: 28.09.2006
(51) Int. Cl.: A61K 9/16, A61K 9/14, A61K 31/192

(54) **Rapidly solubilising formulation of non-steroidal anti-inflammatory drugs**
Schnell lösliche Formulierung von nichtsteroidalen Antiphlogistika
Formulation solubilisée rapidement des anti-inflammatoires non stéroïdiens

(43) Date of publication of application: 02.04.2008
(73) Proprietor: Losan Pharma GmbH, 79395 Neuenburg (DE)
(72) Inventor: Gruber, Peter, Dr., 79249, Merzhausen (DE); Kraahs, Peter, Dr., 79189, Bad Krozingen (DE)
(74) Representative: UEXKÜLL & STOLBERG

(56) References cited:
- WO-A-99/09988
- WO-A1-00/13672
- WO-A2-01/41733
- DE-A1- 19 624 607
- US-A- 5 969 181
- US-B1- 6 171 617

## Description

### FIELD OF THE INVENTION

This invention relates to solubilized non-steroidal anti-inflammatory drugs (NSAIDs), in particular in the form of a granulate, pharmaceutical dosage forms comprising the same as well as a process for producing solubilized NSAIDs and NSAID granulates.

### BACKGROUND OF THE INVENTION

Non-steroidal anti-inflammatory drugs (NSAID) have anti-inflammatory analgesic activity and are widely used for the treatment of pain. A common disadvantage of this group of drugs is their poor solubility. However, water solubility is crucial for absorption and hence bioavailability of a drug; poor solubility results in an undesirable delay in the onset of activity. Especially in the treatment of pain a fast onset of action is required and highly desired.

Furthermore, the solubility of NSAIDs is highly pH-dependent. NSAIDs usually dissolve only at pH values above 6.5 and thus the active ingredient is absorbed merely in the intestinal tract but not in the stomach. NSAIDs which have acid groups in their chemical structure form sticky agglomerates upon contact with the acid gastric juice. Since the pH in the upper-most part of the intestine (duodenum) lies predominantly between 5 and 6 the active ingredient is absorbed mainly in lower parts of the intestine which causes a further delay. Additional delays may be caused by variations of pH conditions in the intestinal tract due to physiological reasons.

Numerous attempts were made to accelerate the onset of action through pharmaceutical measures such as micronization of the active ingredient or the development of fast disintegrating film-coated tablets. However, such attempts did not improve the situation significantly because the onset of action is mainly dependent upon the pH in the intestinal tract.

A clear improvement was achieved by the use of NSAID salts with good water solubility such as naproxen sodium.

DE 44 10 470 A1 discloses pharmaceutical compositions comprising naproxen and arginine in a molar ratio of 1:0.8 to 1:1.5. These compositions are said to result in an accelerated onset of the analgesic activity. They may additionally comprise sodium or potassium hydrogen carbonate in order to improve the rheological properties.

WO 03/061630 discloses self-dispersing or self-emulsifying tablets containing an active substance such as naproxen, an active lipophilic substance, a surfactant and a lipid. The tablets are said to spontaneously form a microemulsion upon contact which water and to improve or facilitate the distribution of a lipophilic drug in the gastrointestinal tract.

CA 2 363 528 is directed to naproxen containing tablets having a fast dissolution rate. The tablet comprises naproxen sodium and spray-dried mannitol.

WO 97/18245 discloses ternary inclusion complexes of naproxen or a pharmaceutically acceptable salt thereof, unsubstituted or substituted beta-cyclodextrin and a hydroxylamin. These ternary complexes are said to have a better taste when compared to binary complexes of naproxen sodium and beta-cyclodextrin at equivalent pH. In addition the complexes are said to increase the water solubility of the drug.

US 6,171,617 B1 relates to a clearly dissolving ibuprofen effervescent formulation and a process for the preparation of this formulation.

US 5,969,181 A1 teaches a process for preparing salts of pharmaceutical active substances which have acidic groups by reacting the carboxylic acids with a base in the melt, wherein the acids are reacted with at least the stoichiometric amount of a base in an extruder.

WO 99/09988 A1 teaches pharmaceutical compositions containing the enolic carboxamide type anti-inflammatory agent meloxicam that exhibit improved wettability, aqueous solubility, dissolution behaviour over a broad range of pH, and that are prepared by crystal structure modification of the drug through dry or wet mechanical homogenization.

WO 01/41733 A2 teaches a compressed tablet composition comprising: a granular component comprising a plurality of solidified melt granules of a NSAID having a melting point in the range of 30 to 300°C and incorporating a disintegrant uniformly dispersed therein.

Moreover, WO 00/13672 A1 teaches solid dose nanoparticulate naproxen formulations having high rates of dissolution.

EP 1 679 301 A1 pertains to esters of nonsteroidal anti-inflammatory drugs and oligo(3-hydroxybutyric acid). The oligomers of 3-hydroxybutyric acid are said to eliminate irritation of the gastrointestinal tract mucosa by NSAIDs and to facilitate the penetration of the drug through cell membranes. Furthermore, the esters are said to show a rapid drug transportation through the gastrointestinal tract and reduced drug toxicity.

US 5,183,829 relates to oral liquid compositions of NSAIDs which are said to demonstrate good reproducible distribution in gastric juice and better absorption of active ingredient into the subject. The compositions contain one or more NSAIDS dissolved in a glycol-polyol-alcohol vehicle along with one ore more selected dispersing agents such as polyvinylpyrrolidone.

None of the known compositions is complete satisfying. There is still a great demand for NSAID dosage forms which achieve a rapid onset of action and which can be produced at low costs.

### SUMMARY OF THE INVENTION

It is an object of the invention to provide a novel and more economic process for producing solubilized non-steroidal anti-inflammatory drug (NSAID) forms.

It is further object of the invention to provide a novel process that facilitates production of granulates in a very efficient way for solubilized NSAIDs forms, especially of the naproxen.

It is another object of the invention to provide novel granulates and other pharmaceutical dosage forms, especially oral dosage forms, on the basis of NSAID that provide a rapid increase of the blood level and a rapid onset of the analgesic action.

In accordance with these objects, the process of claim 1 for producing a solubilized NSAID, preferably as granulate, is disclosed and claimed with the steps of: providing a mixture comprising solid NSAID and a first base which is selected from the group of bases having a pH value of at least 11 in water as 0.1 molar solution or dispersion, and reacting the NSAID and the base in essentially the dry state at a temperature of from 20°C to 90°C. This process results in the formation of solubilized NSAID which is directly further processable without drying. Additionally, a novel solubilized NSAID granulate obtainable by said process is provided as well as novel pharmaceutical dosage forms comprising said granulate.

### DETAILED DESCRIPTION OF THE INVENTION

It has surprisingly been found that a solubilized NSAID can be directly obtained in one step by reacting a NSAID with a base in essentially dry state. Moreover, the obtained solubilized NSAID usually needs not be dried but is suitable for direct use or further processing, for example, for forming tablets. The solubilized NSAID of the present invention has preferably the form of a granulate.

In contrast thereto, conventional preparation and granulation of NSAID salts is a multi-step process usually including preparation of the salt in an aqueous medium by dissolution of NSAID and a base, separation therefrom, drying and granulation of the salt, if possible, and drying of the granulate. While granulation of these known salts is difficult to achieve, solubilized NSAID granulates can be obtained without difficulties in accordance with the process of the invention.

Furthermore, the process of the invention permits incorporation of water soluble excipients into the reaction mixture, and it has surprisingly been found that NSAID and NSAID granulates obtained in this manner, both in the form of the pure NSAID salts and in the form of physical mixtures with water soluble excipients, are superior concerning their physico-technological properties like flowability and compressibility. Without wanting to restrict the scope of the present invention, it is believed that these advantages are at least in part due to the presence of different polymorphic and/or amorphous forms; where more than one base is used, mixed crystals might also be formed.

Additionally, the process of the invention and the properties of the obtained granulate can be varied to a large extent, as desired, by the selection and combination of bases, the incorporation of water soluble excipients and the amount of added water.

The process of the present invention comprises the steps of:
(i) providing a mixture comprising solid NSAID and a first base and
(ii) reacting said NSAID and the base in essentially dry state.

In the scope of the present invention, the term "solubilized NSAID" means water-soluble forms of non-steroidal anti-inflammatory drugs (NSAIDs) wherein at least a part of the NSAID is present in salt form, i.e. a NSAID compound which has been converted into a better soluble form by direct reaction with a base in solid form in essentially the dry state. Preferably essentially all of the NSAID is present in form of a salt.

The active ingredient is selected from the family of NSAIDs which contain at least one carboxylic group. The NSAIDs of the invention comprise at least two aromatic rings in their structure. These rings can be carbocyclic or heterocyclic rings and can form a condensed ring system such as the naphthalene ring system. The NSAIDs are preferably selected from the groups of arylcarboxylic acids and/or arylalkanoic acids. Preferred arylcarboxylic acids are diflunisal, flufenamic acid, mefenamic acid and niflumic acid. Preferred arylalkanoic acids are arylacetic acids, such as sulindac, indomethacin, tolmetin, diclofenac; arylpropionic acids, such as flurbiprofen, fenbufen, fenoprofen, tiaprofenic acid, ketoprofen and naproxen. Most preferred NSAIDs are naproxen, acemetacin, fenbufen, indomethacin, tolmetin and mixtures thereof, in particular naproxen.

The first base used in the process of the present invention is selected from the group of bases which have a pH value of at least 11 in water as 0.1 molar solution or dispersion.

Preferably the first base is selected from sodium hydroxide, potassium hydroxide, sodium carbonate, potassium carbonate, sodium glycinate, potassium glycinate and tribasic sodium and potassium phosphates and mixtures thereof.

The term "tribasic sodium and potassium phosphates" encompasses trisodium phosphate, tripotassium phosphate, disodium monopotassium phosphate and monosodium dipotassium phosphate, including hydrates thereof; preferred are trisodium phosphate and tripotassium phosphate.

According to a preferred embodiment of the present invention, NSAIDs may be reacted with two or more different bases. The mixture may thus contain at least a second base and optionally further bases. The second base and optional further bases may be selected from the bases mentioned above. In addition, any base which has a pH of at least 7.5, more preferably of at least 9 in water as 0.1 molar solution or dispersion is suitable as a second or further base. Examples of pharmaceutically acceptable bases that are suitable for use as second or further base include sodium hydroxide, potassium hydroxide, sodium carbonate, potassium carbonate, sodium glycinate, potassium glycinate, tribasic sodium and potassium phosphates, disodium hydrogen phosphate, dipotassium hydrogen phosphate, trisodium citrate, tripotassium citrate, disodium citrate, dipotassium citrate, disodium tartrate, dipotassium tartrate, disodium malonate, dipotassium malonate, disodium succinate, dipotassium succinate, disodium malate, dipotassium malate, sodium hydrogen carbonate, potassium hydrogen carbonate, sodium acetate, potassium acetate, sodium propionate, potassium propionate, N-methylglucosamine, arginine and lysine.

Preferably, the second or further base is selected from the group consisting of sodium hydroxide, potassium hydroxide, sodium carbonate, potassium carbonate, sodium glycinate, potassium glycinate, tribasic sodium and potassium phosphates, trisodium citrate, tripotassium citrate, N-methylglucosamine, arginine and lysine.

In one preferred aspect, the reaction mixture may thus comprise two or more bases selected from the group consisting of sodium hydroxide, potassium hydroxide, sodium carbonate, potassium carbonate, sodium glycinate, potassium glycinate and tribasic sodium and potassium phosphates.

In another preferred aspect, the reaction mixture may comprise at least one base selected from the group consisting of trisodium citrate, tripotassium citrate, in addition to one or more bases selected from the group consisting of sodium hydroxide, potassium hydroxide, sodium carbonate, potassium carbonate, sodium glycinate, potassium glycinate and tribasic sodium and potassium phosphates.

The use of potassium carbonate as a base has the advantage that the solubilization reaction can usually be carried out under particularly mild conditions without cooling. Moreover, when NSAIDs are reacted with an at least equimolar amount of carbonate, no weight loss is observed, i.e. no carbon dioxide is released; the carbonate is apparently converted to the corresponding hydrogen carbonate.

The use of hydrogencarbonates and in particular sodium hydrogen carbonate as a base in the manufacture of the solubilized NSAID granulate according to the present invention is generally not preferred since they thermally disintegrate thereby forming sodium carbonate, CO₂ and water.

However, the presence of hydrogen carbonate which is formed by reaction of carbonate in the granulate is advantageous since it improves and stabilizes the dissolution of the granulate in gastric juice. Surprisingly, it has been found that such granulates are superior to simple physical mixtures of NSAIDs salts and hydrogen carbonate which may be due to the fact that hydrogen carbonate is formed *in situ* by reaction of the NSAIDs with carbonates and thereby forms an intimate mixture with the NSAIDs salts. Such a mixture can not be achieved by simple mixing of NSAIDs salts and hydrogen carbonate.

In accordance with a further preferred embodiment, the reaction mixture may comprise sodium hydroxide and/or potassium hydroxide as the sole base. Preferably, the hydroxide or hydroxides and the NSAID may be used in equimolar amounts in this embodiment. Although the sodium and/or potassium salts of NSAIDs should theoretically be formed in this solubilization reaction, improved granulates are obtained which differs significantly from the conventionally obtained salts in various properties. Moreover, the present process avoids the difficulties to granulate the conventionally obtained salts.

A further preferred embodiment of the invention concerns processes, in which a potassium-containing base or bases, such as potassium carbonate, are exclusively used. Preferably, the base or bases and the NSAIDs are used in approximately equimolar amounts. Potassium NSAIDs are not available on the market.

The present invention enables the partial or complete solubilization of the NSAIDs, as desired. The extent of solubilization largely depends upon the amount base utilized. In general, it is preferred to completely solubilize the NSAID. In the case of sodium and potassium carbonate, 1 mole is generally sufficient to completely solubilize 2 mole NSAID. On the other hand, the base or bases can be utilized in excess, the unreacted amount of base remains in the granulate as an excipient.

Generally the total amount of base utilized is in the range of 0.5 to 4 mole, preferably 0.7 to 4 mole, more preferably 0.8 to 3 mole, even more preferably 0.8 to 2.5 mole, most preferably 0.8 to 1.5 or 0.9 to 2.5 mole per mole of NSAID. The bases should be present in such amounts that the pH of a quantity of granulate corresponding to 2 mmole NSAID is between 6 to 12, preferably 7 to 10, when placed in 100 ml water. Most preferably, the amount of bases utilized will usually be about 0.7 to 1.2 mol, for example 0.95 to 1.1, per mole of NSAID, especially when only strong bases (i.e. bases having a pH of at least 11 when dissolved or dispersed in water in a concentration of 0.1 M) are used.

Trisodium citrate, tripotassium citrate, if present, are preferably used in a total amount of 0.05 to 0.7 moles, more preferably 0.1 to 0.5 moles, most preferably 0.1 to 0.3 moles, for example about 0.2 moles, per mole of NSAID.

While strong bases are preferably admixed in solid form with the NSAIDs, other bases such as glycinate, in particular sodium glycinate and potassium glycinate may also be prepared *in situ* by reacting glycine with a suitable base, preferably with a sodium and/or potassium base such as sodium hydroxide, potassium hydroxide, sodium carbonate or potassium carbonate, preferably sodium hydroxide and/or potassium hydroxide. It is preferred to react glycine with the base before adding the NSAIDs and any further components.

The invention also concerns solubilized NSAID granulates obtainable by the above processes. Particularly preferred are solubilized NSAID granulates comprising a mixed sodium and potassium salt of the NSAID. Particularly preferred is a solubilized NSAID granulate comprising a mixed sodium and potassium salt of NSAID.

In the following particularly preferred embodiments of the process of the present invention will be described. According to one aspect of the present invention there is provided a process for producing solubilized NSAID, preferably in the form of a granulate, which comprises the steps of: providing a mixture comprising solid NSAID and a first base selected from the group consisting of sodium hydroxide, potassium hydroxide, sodium carbonate, potassium carbonate, sodium glycinate, sodium glycinate monohydrate, N-methylglucosamine, potassium glycinate and tribasic sodium and potassium phosphates and mixtures thereof, and reacting the NSAID and the base in essentially dry state.

The mixture preferably comprises from 0.5 to 1.5 mole, preferably 0.8 to 1.2 mole, most preferably 0.9 to 1.2 mole of the first base per mole NSAID. In addition to the first base the mixtures may comprise other basic compounds. Preferably the total amount of basic compounds is at least 0.8 mole, preferably 0.9 to 1.5 mole of basic compounds per mole NSAID. The mixture of basic compound preferably comprises at least 0.8 mole of the first base per mole of NSAID.

The amount of base or bases is preferably adjusted in such a way that an amount of solubilized NSAID granulate corresponding to 2 mmole NSAID has in 100 ml water a pH-value of 6 to 12 and preferably of 7 to 10.

A particularly preferred embodiment is a process according to the present invention, wherein the first base is selected from the group consisting of sodium hydroxide, potassium hydroxide, potassium carbonate, sodium glycinate or potassium glycinate. The preferred bases are sodium hydroxide, potassium hydroxide and potassium carbonate, sodium carbonate.

In a further preferred embodiment of the process according to the invention the mixture to be reacted comprises two or more, preferably 3 basic compounds. More preferably, the reaction mixtures comprises a first base which is selected from the group consisting of sodium hydroxide, potassium hydroxide, potassium carbonate, sodium carbonate, sodium glycinate and potassium glycinate, and a second base which is selected from the group consisting of tribasic sodium, tribasic potassium phosphates, tri sodium-, tri potassium citrate, lysine, arginine, physiological safe organic amines like meglumine. More preferably the first base is selected from the group consisting of sodium hydroxide, potassium carbonate, sodium glycinate and potassium glycinate, and the second base is selected from the group consisting of potassium hydroxide, sodium carbonate and tribasic sodium and potassium phosphates.

In yet another preferred embodiment of the process according to the invention the reaction mixture comprises at least one base having a pH of 7.5 to < 11, preferably 7.5 to 10 as 0.1 M aqueous solution or dispersion. This base is preferably selected from the group consisting of trisodium citrate, tripotassium citrate, arginine, meglumine and lysine.

In a further preferred process according to the present invention, the reaction mixture comprises at least one sodium-containing base and at least one potassium-containing base. The sodium-containing base(s) and potassium-containing base(s) are preferably present in a molar ratio of 1:20 to 20:1, more preferably 1:9 to 9:1. These bases are preferably selected from hydroxide-containing bases and carbonate-containing bases.

According to a particularly preferred embodiment, the reaction mixture comprises sodium hydroxide together with potassium hydroxide or potassium carbonate as the one or more basic compounds, more preferably at least 0.5 mole and even more preferably at least 0.9 mole sodium hydroxide per mole of NSAID.

According to another particularly preferred example of the process according to the present invention the reaction mixture comprises potassium carbonate together with sodium carbonate or sodium hydroxide as the one or more basic compounds, more preferably at least 0.75 mole and even more preferably at least 0.85 mole potassium carbonate per mole NSAID.

According to the present invention, the NSAID and said one or more basic compounds are reacted in essentially the dry state. As used herein, the term "in essentially the dry state" or "in essentially dry form" preferably means that the process is carried out in the absence of quantities of free water exceeding the quantity required for forming hydrates by more than 2 mole, preferably 1 mole, per mole of NSAID, i.e. the mixture preferably comprises water in an amount which does not exceed the amount required for forming solid hydrates by more than 2 mole per mole of NSAID. More preferably, free water is not added in quantities exceeding the quantity required for forming hydrates by more than 0.5 moles per 1 mole of NSAID, or free water is added only in quantities required for forming hydrates. Most preferably no water is added at all. Traces of water may be present due to the fact that the starting materials of the reaction, i.e. NSAIDs, bases and optional excipients, are usually not dried prior to use and may therefore comprise traces of water.

In particular, the mixture comprises less than 2.5 moles of water per mole of NSAID, preferably 0.1 to 2, more preferably 0.1 to 1.2 mole of water per mole of NSAID. In another preferred embodiment, the process is carried out in the absence of more than 1 mole, preferably 0.5 moles, of free water per mole of NSAID or even in complete absence of free water.

The addition of water can accelerate the reaction and/or can convert the solubilized NSAID into a less hygroscopic hydrated form. In particular, reaction products e.g. of naproxen and one or more sodium-containing bases usually form stable hydrates containing up to about 2 moles of water per 1 mole of naproxen. The process of the present invention has the advantage that the obtained solubilized NSAID or NSAID granulate is a solid, dry product which does usually not require drying before use or further processing.

For instance, the reaction of 0.95 mole of sodium hydroxide and 0.1 mole of potassium hydroxide in the presence of 1 mole of water soluble excipient and 0.8 mole water per mole of NSAID results in solubilized naproxen-hydrate which does not require further drying.

Also, if for example one mole of naproxen is intensively mixed with 0.95 mole sodium carbonate, 0.1 mole potassium carbonate, 1 mole glycine and 0.3 mole potassium chloride and warmed to about 50 °C, followed by the addition of 1.1 mole water per 1 mole naproxen, then this gives a free flowing, entirely dry, highly water soluble naproxen compound that can be processed further to tablets. However, if the naproxen is to be combined with a moisture sensitive active ingredient or excipient it may be advantageous to dry the naproxen compound before further processing.

If water is added directly after the dry mixing of the components, the mixture does not need to be warmed and converts without problem into the described highly water soluble dry NSAID granulate. If in contrast a mixture of one mole of NSAID is warmed with 0.9 moles of potassium carbonate and 0.15 moles of potassium hydroxide, a highly water soluble, fine NSAID granulate forms at a temperature of about 50 to 60°C within short time, e.g. about 1 hour. If per mole of NSAID 0.3 moles of water (about 1.5%) are added with stirring, the mixture surprisingly converts within 20 minutes into a highly water soluble NSAID granulate. Nevertheless, the mass remains surprisingly a solid powder or as a fine granulate.

According to a further preferred embodiment of the present invention, small amounts of a non-aqueous liquid selected from the group consisting of aliphatic C₁-C₄ alcohols, such as ethanol and in particular isopropanol, acetone and mixtures thereof are used in the process of the invention. The amounts of non-aqueous liquid utilized should preferably not exceed 0.25 moles, and more preferably not exceed 0.1 moles per mole of NSAID, and the total amount of free water and non-aqueous granulation liquid preferably does not exceed 2.5 moles per mole of NSAID. According to another preferred embodiment the reaction is carried out essentially in the absence of water but in the presence of a non-aqueous liquid as defined above. In a third preferred embodiment the reaction is carried out essentially in the absence of water and non aqueous granulation liquids by warming the reaction mixture, whereby most of the heat is formed by the exothermic solubilization process.

If a non-aqueous liquid is used in the solubilization process, it is preferred to use sodium carbonate, potassium carbonate or a mixture thereof as the first base, more preferably 0.8 to 1.2 mole of potassium carbonate and 0 to 0.4 mole of sodium carbonate per mole of NSAID.

The reaction mixture preferably also comprises one or more pharmaceutically acceptable excipients, especially excipients that are conventionally used in oral dosage forms, such as fillers, binders, disintegrants, glidants and anti-precipitation agents. These excipients can be added to the mixture of NSAID and base prior to reacting said NSAID with the one or more bases or after the reaction. Incorporation of excipients into the reaction mixture may, for example, improve the flow properties, reduce the hygroscopicity, improve the tabletting properties and improve the dissolution rate of the granulate and the tablets.

Preferred fillers are water-soluble, neutral to acidic substances having a pH of 5 to 7 as 0.1 M aqueous solution or dispersion, for example sugars such as saccharose; hexoses such as isomalt, sorbitol, mannitol, xylitol and maltitol; salts such potassium chloride, potassium sulfate, potassium acetate, sodium chloride, sodium sulfate, and magnesium chloride; polymeric compounds, such as non-crosslinked polyvinylpyrrolidiones, e.g. Povidone K25 to K90, cellulose derivatives such as hydroxypropyl methylcellulose, low substituted hydroxypropylcellulose, α-cyclodextrin, β-cyclodextrin, γ-cyclodextrin, starches (e.g. maize starch), microcrystalline cellulose, polyethylene glycols having molecular weight of 200 to 20000 (e.g. PEG 6000); urea, glycine, glycerol, propylene glycol and mixtures thereof.

Particularly preferred are neutral and water-soluble excipients exhibiting a pH in water of about 7 and a solubility in water at 37°C of at least 5% (w/w), such as potassium chloride, mannitol, isomalt, polymeric compounds, non-crosslinked polyvinylpyrrolidiones, cellulose derivatives, microcrystalline cellulose, tensides, sodium laurylsulfate, saccharose palmitate, glycine and mixtures thereof, more preferably glycine and/or potassium chloride.

Glycine facilitates solubilization of NSAIDs and improves the compressibility and solubility of the obtained granulate. As described above, glycine may form glycinate upon reaction with a base. Glycine is therefore preferably used in excess to the amount which reacts with the base. Preferably, the reaction mixture contains 0.3 to 2 mole, more preferably 0.2 to 1.5 mole, even more preferably 0.7 to 1.3 mole and most preferably 0.4 to 1 mole of glycine per mole of NSAID.

Preferred disintegrants include cross-linked polyvinylpyrrolidone, cross-linked sodium carboxymethylcellulose and sodium carboxymethylstarch.

Preferred glidants are silicon dioxide and talc.

Anti-precipitation agents are particularly preferred excipients which may be incorporated in the reaction mixture. Under the acidic conditions in the stomach the NSAID salts of the solubilized NSAID granulates may be converted back to the corresponding acid form of the NSAID which forms instable supersaturated solutions. Suitable as anti-precipitation agents are all substances that are capable of stabilizing these supersaturated solutions of NSAIDs and/or which delay the precipitation of NSAIDs under acidic conditions.

Preferred anti-precipitation agents are protective colloids, tensides, gelatine, polyvinyl alcohol, hydroxypropylmethylcellulose, hydroxyethylcellulose, non-crosslinked polyvinylpyrrolidone, sodium lauryl sulfate, magnesium lauryl sulfate, ascorbylpalmitate, saccharose fatty acid esters, such as saccharose monopalmitate, saccharose monostearate and in particular tensides having an HLB ratio > 12.

The use of tensides is particularly advantageous if the solubilized NSAID granulate contains carbonate or hydrogen carbonate since carbonates and hydrogen carbonates release carbon dioxide upon contact with the acid environment in the stomach. In the presence of a tenside the carbon dioxide forms a foam and thus a basic micro-environment is generated around the tablet or granulate which delays penetration of further gastric juice. This enables a particularly rapid resorption in the duodenum and a particularly rapid increase of the blood levels.

Another particularly preferred anti-precipitation agent is non-crosslinked polyvinylpyrrolidone (PVP). PVP is preferably incorporated into the reaction mixture in a PVP/NSAID weight ratio of about 0.01 to 0.3:1, more preferably about 0.05:1 to 0.2:1 and most preferably about 0.1:1 to 0.15:1.

Lubricants such as magnesium stearate and stearic acid may also be incorporated into the reaction mixture but are preferably added to the solubilized granulate after reaction.

The reaction mixture may preferably contain up to about 50%, more preferably up to about 40% and most preferably up to about 30% (w/w) of excipients, based on the total weight of the mixture. If present, the amount of excipients is usually at least about 1% (w/w). However, the reaction mixture can also be completely free of excipients.

Water-soluble excipients are preferably used in an amount of up to 20 mole, preferably 0.25 to 4 mole, more preferably 0.5 to 1.5 mole per mole of NSAID.

It is generally preferred that the reaction mixture comprises 1 to 20 %, preferably 1 to 15 %, more preferably 1 to 9 %, and most preferably 4 to 7% by weight of polymeric compounds. According to another preferred embodiment the mixture comprises 0 to 4 % by weight and even more preferably 0 to 3 % by weight of polymeric compounds. In particular, the reaction mixture comprises 0 to 9 % by weight, preferably 1 to 9 % by weight of polyvinylpyrrolidone or cellulose ester like hydroxyethylcellulose, hydroxymethylpropylcellulose, gelatine, xanthan. These and other weight percentages as specified herein are based on the total weight of mixture if not indicated otherwise.

The solubilization reaction can be carried out under mild conditions without cooling or heating. Preferably the reaction of NSAID and base is carried at a temperature of from 20 to 150 °C, preferably 20 to 120°C, more preferably 20 to 90 °C or 20 to 65 °C but higher temperatures are also possible. If a non-aqueous liquid is used, the reaction is preferably carried out at a temperature of from 40 to 60°C, preferably 50 to 60 °C.

The process in accordance with the present invention is usually slightly to strongly exothermic. However, it may sometimes be helpful to heat the reaction mixture, for example, to about 35 to 50°C to start or accelerate the reaction. When hydroxides or mixture of hydroxides are used, however, cooling may rather become necessary or desirable, especially in the case of large batches.

It is preferred to use NSAIDs and bases in the form of particles having small grain sizes in order to accelerate the solubilization reaction. Advantageously, at least 95% of the NSAID particles have a particle size of less than 100 µm and/or at least 95% of the base particles have a particle size of less than 150 µm, preferably less than 75 µm, as determined by sieve analysis. Preferably, NSAIDs have a mean particle size of less than 60 µm. Particle sizes are determined according to DIN 66144.

The process of the present invention can, surprisingly, be carried out in any devices conventionally used in the manufacture of pharmaceutical oral dosage forms, in particular mixing vessels such as wet mixing vessels, wet mixing vessel provided with chopper and impeller compactors, vacuum mixing vessel with impeller and chopper, fluid bed granulators, vacuum granulators and extruders. The mixing vessel may comprise means for cooling and/or heating the mixture in said vessel.

The solubilized NADS may be further treated e.g. by comminuting, granulating, drying, compressing, compacting and/or filling into sachets or stick packs. As has been explained above, in some cases NSAID compounds can bind water by the formation of hydrates such that the product can be further processed for example to tablets without drying even if water is added or formed in the reaction.

The reaction time depends upon the particular production method utilized, the election of base or bases, the temperature, the particle sizes, the presence of excipients, the amount of added water etc. Generally, the reaction time can range from a few seconds up to several hours. The progress of the solubilization reaction can be checked by removing samples and testing their solubility in water. At the beginning of the reaction the poorly soluble NSAIDs swim at the surface of the water. Progress of the solubilization reaction improves the wetting of the NSAID crystals by the water. Solubilization is completed when no NSAID remains undissolved. If the granulate does not contain poorly soluble excipients such as fillers, a clear solution is generally formed after completion of the solubilization.

When no heating and cooling is required, NSAIDs and the base (or bases) can simply be placed in a conventional mixing vessel and mixed until the desired granulate is obtained. For example, if one mole naproxen is intensively mixed with 0.4 mole of sodium carbonate, 0.8 mole of potassium carbonate and 0.3 mole of water, the temperature of the reaction mixture increases to about 40 °C, and the solubilization of the NSAID is completed within about 30 minutes. Drying of the solubilized naproxen is not necessary.

Also preferred is a solubilized NSAID granulate as obtainable when 1 mole of NSAID is reacted at a temperature of from 20 to 65°C in admixture with about 0.9 mole potassium carbonate, about 0.1 mole of Na₂CO₃, 5% by weight of saccharose palmitate, based one mole of NSAID, and up to 0.3 mole of water, preferably without any water.

Solubilized granulates of NSAIDs according to the present invention can also be prepared in a suitable wet mixing vesse. A preferred composition for processing in a wet mixing vessel comprises from 0.8 to 1.2 mole potassium carbonate and 0 to 0.4 mole NaOH and/or Na₂CO₃ per mole of NSAID, and optionally further excipients such as povidone K25 and/or tensides such as sodium lauryl sulfate or saccharose monostearate.

The solubilization is preferably performed at a temperature within the range of 50 to 60 °C with intensive stirring and without the addition of water. It was surprisingly found that the whole solubilization process can be performed practically in the dry state at temperatures between 40 and 55 °C, i.e. below the melting point of NSAIDs, in the absence of water. The reaction can be accelerated by the addition of traces of non-aqueous liquids such as ethanol, acetone and in particular isopropanol in a magnitude of 0.25 mole per mole NSAID. For instance, about 6 mg of solvent are used per mole of naproxen (206 mg). In this case the reaction will be completed within 30 minutes. Under vacuum drying of the reaction product can be achieved within less than 5 minutes.

Said mixing vessels may comprise means for cooling and/or heating the mixture in order to simplify control of the reaction of the NSAIDs with the basic compounds. Preferably, the mixing vessel or wet mixing vessel is provided with an impeller and chopper.

Following the treatment in a mixing vessel, the solubilized NSAIDs can be further treated e.g. in a fluid bed granulator in order to enlarge the granulate structure.

For example, 0.4 mole sodium hydroxide, 0.4 mole sodium carbonate, 0.3 mole tripotassium phosphate, 0.8 mole glycine and 1 mole water are stirred for 10 minutes in a mixing vessel. 1 mole naproxen and 5% (w/w) povidone K25 are added and the whole mixture is treated with impeller and chopper for about one hour. Throughout the reaction the mixture remains dry and flowable. After about one hour, the solubilization is completed. The end of the reaction is indicated by the fact that the product readily dissolves in water. For instance, a quantity of granulate corresponding to 400 mg naproxen completely dissolves in water within less than 30 seconds and forms a clear solution. The granulate can then be transferred into a fluid bed granulator and be treated for example with a 7% (w/w) aqueous solution of povidone K90 to give a coarser granulate, which can easily be compressed to tablet cores.

Mixtures comprising non-aqueous liquid and/or carbonates can advantageously be processed in a vacuum granulator. For instance, if potassium containing bases are used, it is preferred to use bases which do not produce water in the neutralization reaction with the NSAID, such as potassium carbonate and/or potassium glycinate, and to use an non-aqueous liquid, preferably ethanol or isopropanol. The non-aqueous liquid is preferably added in an amount of 0 to 0.25 mole, preferably 0 to 0.1 mole per mole of NSAID. Vacuum granulation results in the formation of dry, solubilized NSAID granulates which are free flowing and which do not require further drying.

According to an alternative embodiment of the invention the mixture of NSAIDs, base or bases and optional excipients is compacted in a suitable compactor. The compacted product mixture is preferably comminuted after the reaction in order to obtain a granulate. Compaction can be effected with conventional compactors, for example, a roller compactor (dry compactor) or by compression the reaction mixture to tablets (slugging). The compacts or tablets can be broken on a suitable screen, for example a rotating screen. It turned out, completely surprising for a person skilled in the art, that for instance during the compaction of 1 mole naproxen with 1.05 mole potassium carbonate the mechanical stress occurring during compaction and the heat generated thereby is sufficient to obtain a completely dry solubilized granulate which is completely water soluble.

In a particularly preferred embodiment of the process according to the present invention, said mixing vessel is an extruder, more preferably an extruder-granulator, most preferably a twin screw extruder. The extruder preferably comprises 8 to 12 barrels, one or several gravimetric feeders for the active ingredient(s), the alkaline component(s) and excipient(s), screw(s), screw shaft(s), a barrel heater/cooler system, an exit die and/or an extrudate cutter.

The extrusion process is preferably carried out continuously.

An extruder-granulator provides for a free variation of compounding pressure and molding temperature by choice of screw geometry, rotational speed and screw elements to be mounted on the screw shafts. The barrel can be used in a variety of combinations of length according to the intended use, and its temperature can be controlled as desired.

Depending on the rotational speed of the screws, the mean residence time of the material in the extruder-granulator can vary. Generally a residence time of about 30 to 120 seconds is preferred for the extrusion process. The material is usually discharged through an exit die with a diameter of about 0.5 mm to several centimeters. Preferred is a discharge of the mass in form of a cylindrical rope. Depending on the temperature of the extruded mass the material can be milled immediately or after cooling. By use of appropriate nozzles, the solubilized mass can also be extruded in the form of spaghetti type bands, or bands having e.g. a width of 5 cm and a height of 0.5 mm. These bands usually solidify after a few seconds through air cooling and can subsequently be milled to well structured granulates.

For extrusion, the first base is preferably selected from the group consisting of sodium hydroxide, potassium hydroxide, sodium glycinate, potassium glycinate, tribasic sodium phosphates, tribasic potassium phosphates and mixtures thereof. The use of sodium hydroxide and/or potassium hydroxide as first base, preferably together with other bases, is particularly preferred in the extrusion process. The solubilization process can be accelerated by use of sodium hydroxide and/or potassium hydroxide or K₂CO₃, either alone or together with other bases. Reactions with hydroxides are particularly rapid, highly exothermic, produce an equimolar amount of water and accelerate the reaction of co-present bases if present.

A direct reaction (solubilization) of NSAID with potassium hydroxide and sodium hydroxide in solid form in the presence of only 0 to 1 mole of water per mole of NSAIDs has not previously been described in prior art. The heat of neutralization created in this reaction is so high that upon stirring a batch of about 10 kilos the temperature in the mixture may rise to about 100 °C without external heating. In larger batches the temperature may even raise to such an extend that the mixture discolors and decomposition products form. With batches of production size of e.g. 500 kilos, an explosive, non-controllable thermal reaction may occur which results in a complete disintegration of the product and severely endangers the production personnel.

According to the present invention this prior art problem can be overcome by using a continually working extruder granulator for the solubilization reaction of the NSAIDs. Neutralization heat can simply be discharged e.g. by cooling of the barrel of the extruder and/or consumption of thermal energy by melting of the NSAID crystal lattice during extrusion. Such a direct, controlled reaction of NSAIDs with alkali hydroxides in essentially dry state has previously not been envisaged as a possible reaction route by those skilled in the art.

The reaction components, e.g. the NSAID, base or mixture of bases, water and preferably further water soluble excipients are preferably dosed to the extruder by use of gravimetric feeders. The heat of solution of the bases and the water and the heat of neutralization almost result in a spontaneous solubilization of the NSAID and, depending on the cooling employed, the solubilized material is obtained in the form of a granulate or a fluid dispersion which converts into a solid state within seconds upon cooling.

In a typical embodiment of the extrusion process of the present invention, 0.95 mole of sodium hydroxide and 0.05 mole of potassium hydroxide, 0.7 mole glycine, 0.7 mole potassium chloride (as water soluble excipients), 2.5% sodium laurylsulfate, based on one mole of NSAID, and 0.75 mole water are used are used per mole of NSAID.

In a preferred embodiment solid sodium hydroxide pellets of a diameter of approx. 1 mm together with glycine, potassium chloride, sodium laurylsulfate and water are dosed in the extruder where they within seconds form a solution or suspension. This mixture reacts spontaneously with the subsequently dosed NSAID to give a highly water soluble NSAID compound.

For extrusion, the quantity of added water is preferably 0 to 1.5 mole, more preferably 0.6 to 1 mole of water per mole of NSAID. Additional water is formed by the neutralization reaction between NSAID and hydroxides.

Bases having a high solubility in water such as potassium hydroxide can advantageously be dissolved in the water to be added to the reaction. However, the base(s) can of course also be added in solid form. Alkali hydroxides, preferably one or two different alkali hydroxides, are preferably used in a total quantity of up to 1.2 mole per mole NSAID, if desired with addition of other alkaline compounds as disclosed herein. Preferred are mixtures of 0.8 to 0.95 mole sodium hydroxide and 0.02 to 0.3 mole potassium hydroxide or potassium carbonate per mole of NSAID.

It is also preferred to use one or more water soluble excipients in the extrusion process, preferably glycine, potassium chloride, sodium laurylsulfate, urea, hexoses and in particular glycine and/or potassium chloride. The amount of these soluble excipients is 0 to 20 mol, preferably 0.25 to 4 mole and most preferably 0.5 to 1.5 mole per mole of NSAID. Glycine is preferably used in an amount of 0.2 to 1.5 mole, preferably 0.7 to 1.3 mole, more preferably 0.4 to 1 mole of glycine per mole of NSAID, and potassium chloride is preferably used in an amount of 0.3 to 1 mole of per mole of NSAID.

For improving the structure of the granulate and its flow capabilities, it is advantageous to use 0.9 mole potassium carbonate and 0.1 to 0.2 mole sodium hydroxide or Na₂CO₃. The sodium base produces the corresponding sodium salt of the NSAID which is usually more sticky than the potassium salt and thus acts as a binder. A small amount of sodium salt significantly improves the total structure of the extruded granulates. By mixing sodium and potassium bases the physico-technical properties and physico-chemical properties of the solubilized granulate, such as compression properties, sticking properties to the punches, dissolution rate and hygroscopicity, can thus be adjusted in a simple manner.

If predominately sodium containing bases are used for the solubilization, sodium hydroxide is preferred in the extruder process. In this case, water is preferably added in an amount ranging from 0 to 1 mole per 1 mole NSAID. Again free-flowing, dry, solubilized granulates are formed. These granules contain the sodium salts of the NSAIDS which can bind water through hydrate formation. Therefore, these granulates do usually not have to be dried.

The production costs for the solubilized NSAID compounds are very low due to the surprisingly simple production method and by the use of the cheap excipients. Additional savings are realized by avoiding the need for a drying step.

It is unexpected to one skilled in the art that by the selection of excipients and the conditions of the extrusion process very highly compressible solubilized NSAID granulates can be produced directly and at favorable costs.

The initial reaction mixture for producing NSAID granulates of the invention by extrusion preferably contains per 1 mole NSAID: 0.8 mole to 1.2 mole sodium hydroxide, 0 to 0.3 mole potassium hydroxide or potassium carbonate or tripotassium phosphate, 0,2 to 2 mole glycine, 0 to 1 mole potassium chloride, 1 to 4 % sodium lauryl sulfate, based on the weight of 1 mole of NSAID, and 0 to 1,5 mole of water; preferably 0,9 to 1,05 mole sodium hydroxide, 0,05 to 0,15 mole potassium hydroxide or potassium carbonate, 0,4 to 1 mole glycine, 0,25 to 0,7 mole potassium chloride, 1,5 to 3 % sodium laurylsulfate and 0,6 to 1,2 mole water. These granulates are particularly suitable for high performance tabletting presses which produce more than 600.000 tablets per hour. Tablet cores produces from these granules having e.g. a diameter of 10.5 mm show no capping tendency and achieve a hardness of more than 130 N.

Another specific and particularly preferred form of solubilized NSAID granulate according to the present invention is a granulate as obtainable when 1 mole of NSAID is reacted at a temperature of from 20 to 85°C in admixture with about 0.95 mole sodium hydroxide and about 0.05 mole of either potassium hydroxide or potassium carbonate; about 0.5 mole of glycine; about 0.3 mole of potassium chloride, about 2% by weight, referring to one mole NSAID, of saccharose palmitate and optionally up to 1.2 mole, preferably about 0.8 mole water.

The solubilized NSAIDs salts produced by the extrusion process have a higher bulk-density than conventionally produced granulates and as a consequence can be better compressed e.g. into tablets.

In addition, air which is usually introduced into the extruder together with the starting materials is incorporated into extrudate in the form of the small bubbles. The granulate produced thus has a porous structure which additionally improves compression properties. The pores have a size of e.g. 5 to 30 µm. The porous structure is verifiable by raster electronic microscopy.

Fine granulates formed by the above processes can be converted into coarser granulates after completion of the solubilization process e.g. by dry compaction or moist granulation. For instance, the solubilized NSAID granulates obtained in accordance with the present invention may be sprayed with a small amount of aqueous granulation liquid, for example a 5 to 20% (w/w) solution of non-crosslinked polyvinylpyrrolidone such as Povidone K17 to K90, to bind fine particle fractions. The spraying-solution can advantageously contain other customary water soluble or water insoluble excipients which improve the compression properties of the granulate and/or avoid the sticking to the tablet punches.

The solubilized NSAIDs granulate obtained by the process of the present invention is characterized by a number of advantages.

The extremely good flowability, compressibility, solubility and in particular the high dissolution rate of the solubilized NSAID granulates was completely unexpected. Furthermore, they have a high stability in solution and an excellent bioavailability. They have good tabletting properties and can be processed to pharmaceutical dosage forms such as tablets, having high hardness, showing rapid disintegration and dissolution in aqueous media and gastric juice and which show a rapidly onset of analgesic effect.

The solubilized NSAIDs obtained by the process of the present invention have excellent structural characteristics and physico-technical and physicochemical properties. NSAID granulates produces according to the invention, either dissolved in water or swallowed as tablets, give high blood levels of the active ingredient within short time periods.

They are superior to the pure potassium or sodium salts of the NSAIDs and can easily be distinguished from known salts by common analytical methods. The differences are particular prominent if glycine is used as an additive. If, for example, one mole naproxen is reacted with 0.95 mole sodium hydroxide and 0.2 mole glycine, a unique new naproxen-compound is formed which shows dramatic changes in DSC, TGA, powder X-ray and IR. Even traces of glycine have a strong impact on the DSC thermalgram. The former sharp melting peak of sodium naproxen disappears and a broad melting region is visible. The required heat of dehydration is significantly decreased. Apparently a solubilized naproxen sodium/glycine mixed crystal is formed. It is clearly proven by the powder X-ray diffraction pattern that this is not a solidified amorphous mass. No unreacted naproxen is found anymore.

Another aspect of the present invention are pharmaceutical compositions comprising a solubilized NSAID or solubilized NSAID granulate prepared by the process of the present invention. The pharmaceutical composition is preferably a pharmaceutical dosage form, in particular a solid dosage form such as, for example, a tablet, film coated tablet, sugar coated tablet, granulate filled in sachets or stickpacks, capsule or suppository. Preferred dosage forms are tablets and granulates.

This pharmaceutical composition may in addition to the solubilized NSAID or NSAID granulate also comprise one or more basic compounds. Since e.g. tablets are exposed to the acidic gastric juice after ingestion, it is of advantage that the solubilized NSAID salt is protected through further basic adjuvants. These additional basic compounds increase the buffer capacity of the tablets against the hydrochloric acid which is found in the stomach and support the dissolution process of the tablets as well as the formation of supersaturated solutions of dissolved NSAIDS under acidic conditions. The additional basic compounds are preferably selected form the group consisting of sodium and/or potassium hydrogencarbonate, sodium carbonate, potassium carbonate, tribasic sodium and potassium phosphates and mixtures thereof. Particularly preferred additional basic adjuvants are compounds which as 0.1 molar solution have a pH of ≤ 10 such as potassium hydrogencarbonate, sodium hydrogencarbonate, dipotassium phosphates, dipotassium citrates, disodium phosphates, disodium citrates.

These basic compounds are preferably added to the final blend before the tabletting process. They can also be added after the solubilization reaction, e.g. by dosing into the extruder, or by addition to a wet mixing vessel after the solubilization. Potassium hydrogencarbonate and sodium hydrogencarbonate should be added after the solubilization process, e.g. to the final mixture for tablets.

The pharmaceutical compositions may also comprise one or more pharmaceutically acceptable excipients. Generally, all common tabletting excipients can be used, excipients which are usual for NSAID-based compositions are preferred. These are preferably selected from the group consisting of microcrystalline cellulose, which in general increases the hardness of tablets, disintegrants such as cross linked polyvinyl pyrrolidone, lubricants and flowability improving agents such as magnesium stearate, stearic acid, silicon dioxide, talc and in particular highly water soluble excipients which improve the dissolution process of the tablet, such as urea, betain-monohydrate, potassium sulphate, potassium acetate and hexoses such as mannitol and sorbitol.

In addition a small quantities of sweetener and/or aroma can added to the granulate.

The pharmaceutical compositions can also comprise further medicaments, such as antihistamines, decongestants, antacids, analgesics, expectorants, anaesthetic and combination thereof. Preferred medicaments are for example diphenhydramine, chlorpheniramine maleat, brompheniramine maleat, phenylpropanolamine, phenylephridine hydrochloride, pseudoephedrine hydrochloride, acetaminophen, codeine and sodiumascorbate.

Excipients and additional medicaments can be added after the solubilization directly into the mixing vessel, into the granulation vessel or into the final mixture.

The compositions preferably comprise 25 to 1000 mg of NSAID per dosage form. Preferred dosages for naproxen or fenbufen are 50, 100, 200, 400, 600 and 800 mg, for diflunisal 500 and 1000 mg; for flurbiprofen 150, 200 and 300 mg; for indomethacin 25, 50 and 100 mg, ketoprofen 25, 50, 75 mg; mefenamic acid 500 and 1000 mg.

Generally, the final pharmaceutical dosage forms preferably contain 0 to 95%, more preferably 3 to 40% and most preferably about 5 to 80% (w/w) of excipients, based on the total weight of dosage form.

Tablets can be uncoated or coated, e.g. with a sugar coating and/or film coating. As coating materials, all common types of sugars and film coating materials are suitable. The amount of coating, related to the tablet cores, is preferably within a range of from 15 to 50 % by weight for sugar coatings and from 1 to 10 % by weight, preferably from 2.5 to 5 % by weight for film coating, based on the weight of the tablet cores.

Tablets or film-coated tablet preferably contain 0 to 40%, more preferably 5 to 15% (w/w) of excipients, based on the total weight of dosage form. Tablet preferably contain a lubricant, for example about 1% (w/w) magnesium stearate.

The NSAID granulates of the present invention can easily be compressed to tablets, preferably at 20 to 25°C and max. 35%, preferably max. 30% relative humidity. The granulates of the present invention unexpectedly give extremely hard tablets, even at high tabletting speeds. For instance, round, biconvex tablets with a diameter of 10.5 mm and a weight of about 350 mg and containing 200 mg solubilized naproxen have hardness of 100 to 180 N. Nevertheless these tablets completely dissolve in a disintegration tester in water at 37 °C within two minutes, or within 2.5 to 3 minutes in a dissolution test in accordance with the European Pharmacopoeia in artificial gastric juice at 37°C and 100 rpm according to the European Pharmacopoeia, 12th Edition, 2005.

Film tablets produced by aqueous coating show a disintegration time of 3 to 4 1/2 minutes. The film tablets are also chemically and physically absolutely stable under stress conditions of 40°C/75% relative humidity in suitable packaging material such as PP-tubes or Alu/Alu-Blister. It was complete unexpected that tablets with this extreme mechanical stability and dissolution rate could be prepared without the use of expensive excipients such as cellulose and super disintegrants.

Although these tablets disintegrate significantly faster than known tablets and rapidly give high blood levels of the active ingredient, they can be produced at significantly reduced production costs.

The NSAID granulates of the present invention are also suitable for filling into tightly closing sachets or stick packs. A granulate containing e.g. 200 mg naproxen dissolves in about 15 seconds in 150 ml water at 20 °C. Due to the low quantity of alkali carbonates (about 150mg), which were added for the solubilization of the NSAID, the taste is excellent and can be hardly differentiated from pure water.

The NSAID granules may be mixed with further excipients before filling them into sachets or stick packs, preferably sweeteners, flavors, water soluble excipients which support the dissolution process of the granules in water, such as the excipients defined above. Granules to be filled into sachets or stick packs preferably contain 0 to 95%, more preferably 5 to 50% (w/w) of excipients based on the total weight of the dosage form.

### EXAMPLES

The invention is further illustrated by the following examples. In the examples, Povidone^{®}K17-K90 denote non-crosslinked polyvinylpyrrolidone and Aerosil denotes a silicon dioxide. NSAIDs and the bases were utilized with the following particle sizes: at least 95% of the NSAID particles had a particle size of less than 100 µm; at least 95% of the base particles had a particle size of less than 150 µm. The tablets manufactured according to the examples, can be coated, if desired, with a sugar coating and/or film coating.

### Examples 1-15

The examples summarized in Table 1 were carried out in a heatable and coolable mixing vessel.

**Table 1**

| **Ex.** | **Composition^{a)}** | **Heating^{b)}** | **Product Temp.^{c)}** | **Remarks** |
|---|---|---|---|---|
| **1** | 0.6 mole NaOH | (ambient) | 81°C | highly exothermic; addition of 0,4 mole water; granular powder, compressible to tablet cores without drying |
| | 0.5 mole KOH | | | |
| | 1.0 mole Naproxen | | | |
| **2** | 0.4 mole NaOH | 38°C | 41°C | addition of 0.5 mole water, granular powder, good compressible to tablet cores |
| | 0.4 mole KOH | | | |
| | 0.4 mole Arginin | | | |
| | 1.0 mole Naproxen | | | |
| **3** | 1.0 mole K₂CO₃, | 65°C | 86°C | addition of 0.2 mole water hygroscopic, fine powder, extremely good compressible, extremely water soluble |
| | 0.1 mole Lysin | | | |
| | 1.0 mole Naproxen | | | |
| **4** | 0.8 mole K₂CO₃, | 40°C | 74°C | addition of 0.2 mole water; flowable granules, very good compressible |
| | 0.3 mole NaOH | | | |
| | 1.0 mole Kefoprofen | | | |
| **5** | 0.6 mole K₂CO₃, | (ambient) | 78°C | plastic mass,; dry powder rapidly formed, good compressible |
| | 0.1 mole NaOH, | 40°C | | |
| | 0.2 mole Na₃PO₄·12H₂O | | | |
| | 1.0 mole Ketoprofen | | | |
| **6** | 0.8 mole Na₂CO₃, | 40°C | 45°C | sticky solid mass; addition of 0.6 mole water after solubilization; dry granulate immediately formed, good compressible |
| | 0.2 mole K₂CO₃ | | | |
| | 0.5 mole urea | | | |
| | 1.0 mole Tolmetin | | | |
| **7** | 0.9 mole NaOH, | (ambient) | 62°C | addition of 0.9 mole water after solubilization; dry granulate immediately formed, good compressible |
| | 0.5 mole glycine | | | |
| | 0.1 mole KHCO₃ | | | |
| | 1% sodium lauryl sulfate | | | |
| | 1.0 mole Indometacin | | | |
| **8** | 0.5 mole NaOH, | (ambient) | 49°C | Addition of 1.0 mole water, granular dry powder immediately formed, good compressible |
| | 0.2 mole Na₂CO₃, | | | |
| | 0.3 mole N-methyl | | | |
| | glucosamine | | | |
| | 1.0 mole Naproxen | | | |
| **9** | 0.2 mole Na-carbonat | 40°C | 48°C | Addition of 0.2 mole isopropanole; dry granulate formed immediately, good compressible |
| | 0.9 mole K₂CO₃, | | | |
| | 0.8 mole Mannit | | | |
| | 1.0 mole Sulindac | | | |
| **10** | 0.2 mole Na-carbonat | (ambient) | 64°C | Addition of 0.4 mole Isopropanole, granular dry powder formed immediately, good compressible |
| | 1.0 mole K-carbonat | | | |
| | 1.0 mole Natrium | | | |
| | hydrogencarbonat | | | |
| | 1.0 mole Indometacin | | | |
| **11** | 0.8 mole K₂CO₃, | (ambient) | 38°C | Addition of 0.1 mole isopropanol before solubilization; solid dry mass |
| | 0.2 mole Na₂CO₃ | | | |
| | 1.0 mole Fenoprofen | | | |
| **12** | 0.7 mole K₂CO₃, | (amient) | 46°C | Addition of 0.2 mole water before solubilization; granular dry powder formed immediately, KHCO₃ added after solubilization, good compressible |
| | 0.2 mole NaOH, | | | |
| | 0.7 mole K2PO4 | | | |
| | 0.5 mole KCl | | | |
| | 1.0 mole Flubiprofen | | | |
| **13** | 0.95 mole NaOH, | (ambient) | 79°C | Addition of NaOH and glycine to 0.8 mole water before mixing with further components; granular powder formed quickly, good compressible |
| | 0.05 mole K₂CO₃, | | | |
| | 0.6 mole glycine, | | | |
| | 0.5 mole beta-Cyclodextrin | | | |
| | 3% saccharose palmitate, | | | |
| | 1.0 mole Naproxen | | | |
| **14** | 1.1 mole NaOH, | (ambient) | 72°C | dispersion of NaOH, KOH and glycine in 1.0 mole water before addition of Fenbufen; solubilized granulate, good compressible |
| | 0.1 mole KOH, | | | |
| | 1.0 mole glycine, | | | |
| | 10% Maltit | | | |
| | 1.0 mole Fenbufen | | | |
| **15** | 0.4 mole NaOH, | (ambient) | 42°C | addition of NaOH, KOH, Trisodium citrate and glycine in 1.6 mole water, addition of further components, granulate, good compressible |
| | 0.1 mole KOH, | | | |
| | 0.5 mole Trisodium citrate, | | | |
| | 1.0 mole glycine, | | | |
| | 8% Mannit | | | |
| | 1% saccharose palmitate | | | |

| | | | | |
|---|---|---|---|---|
| a) Amount(s) of base(s) and excipient(s) indicated in mole per mole NSAID and in % by weight based on the weight of NSAID, respectively; Cit = citrate. b) Temperature to which the mixing vessel was heated; (ambient) means conversion without heating. c) Temperature indicates the maximum temperature of the reaction mixture. | | | | |

All granulates obtained showed complete solubilization of the NSAID utilized and excellent water solubility of the obtained solubilized form. Dissolution rate was measured in a dissolution apparatus, type 2, in accordance with the European Pharmacopoeia by placing 1 g of the granulate into 900 ml water at 37°C at a paddle speed of 100 rpm. The active ingredient dissolved in all cases within 60 seconds. In contrast to the acid form of NSAID utilized, all granulates were wetted immediately and sank to the bottom of the dissolution vessel.

### Example 16

a) 6.9 kg (30 mol) naproxen, 2.25 (30 mol) glycine and 1.2 kg (30 mol) ground sodium hydroxide were mixed in a mixing vessel. Within 20 minutes, the temperature of the vigorously stirred mixture increased to 68°C, while the mixture was transformed into a viscous mass. A sample of 1 g of the mass dissolved in 100 ml water at 37°C within 50 seconds thus indicating that the solubilization was completed. Within 5 minutes, 540 g water were added to the warm mass which was transformed into a coarse granulate within 10 minutes while stirring slowly. After further 15 minutes at about 60°C, the granulate was sieved through a screen having a mesh size of 1.5 mm. The loss on drying of the obtained granulate at 105°C for 30 minutes was 9,9% w/w. Storing of a sample of the granulate in a desiccator at 25°C and 75% relative humidity for 2 months led to an uptake of water of only 0.4% w/w.

### Example 17

230 kg (1000 mol) naproxen were intensely mixed with 16 kg (400 mol) ground sodium hydroxide, 47.7 kg (450 mol) sodium carbonate, 13.8 kg (100 mol) potassium carbonate and 7 kg saccharose monostearate in a mixing vessel. The temperature of the mixture increased to about 50°C, and a slightly tacky granulate was formed within 30 minutes. A sample of 1 g of the granulate dissolved in 100 ml water at 37°C within 25 seconds. The solubilized granulate was transferred into a fluid bed granulator and sprayed with 100 1 water at an inlet air temperature of about 30°C. The water content of the obtained granulate was 8.2% w/w, measured as loss on drying at 70°C within 30 minutes. Storing of the granulate in a desiccator at 25°C and 75% relative humidity for 3 months led to an uptake of 3.2% (w/w) water.

The granulate obtained in the fluid bed granulator was mixed with 1.5% magnesium stearate and compressed into tablets having a diameter of 10.5 mm and containing active ingredient in an amount corresponding to 200 mg naproxen. The tablet hardness was between 50 N and 75 N, and the disintegration time in water was 5.6 minutes.

### Example 18

223.1 kg (970 mol) naproxen and 140 (1013 mol) kg potassium carbonate and 15 kg Povidone K25 were mixed and continuously filled into the funnel of a roller compactor (Bepex roller). Through the action of pressure and heat generation during compaction a mass was formed which was screenable through a 2.5 mm sieve and soluble in water. The sieved product was compressed into tablets in a climatized production room (20-25°C, 20% relative humidity). The tablets had a tablet weight of 340 mg and an active ingredient content corresponding to 200 mg naproxen. The hardness of the round shaped tablets were 50 N, the disintegration time in water at 37°C about 2.2 min.

### Example 19

459,3 kg (1997 mol) naproxen, 84.8 kg sodium carbonate (800 mol), 82.8 kg potassium carbonate (599 mol), 40 kg HPMC, 15mPaxs(2% solution), 9 kg silicium dioxide, 7 kg saccharose monostearate and 16 kg ground sodium hydroxide (400 mol) were introduced into a mixing vessel and mixed vigorously. Within 120 minutes, the temperature increased to 49°C, and the powder agglomerated to a granulate. A sample of 1 g of the granulate dissolved in 100 ml water at 37°C within 30 seconds. The thermosulubilization took place without any added water.

The very fine granulate was transferred into a fluid bed granulator and sprayed with 200 kg of a 9% w/w aqueous solution of glycine to bind powder fractions; the temperature of the inlet air was 35°C. The obtained granulate was nearly dust-free and had a water content of 1.2% w/w (measured as loss on drying at 70°C within 30 minutes). Subsequently, the granulate was mixed with 25 kg microcrystalline cellulose and 10 kg magnesium stearate for 15 minutes, and the mixture was compressed into biconvex tablets having a tablet weight of about 365 mg and an active ingredient content equivalent to 200 mg naproxen. The hardness of the tablets was 40-60 N, and the disintegration time in water, measured in accordance with the European Pharmacopoeia, was 5.0-6.5 minutes.

### Example 20

In the first barrel segment of the twin screw extruder are gravimetric dosed per hour 39.1 kg of a mixture consisting of 38,1 kg NaOH and 1,0 kg silicium dioxide and a powder mixture of 60.0 kg glycine, 25.76 kg potassium chloride, 4,0kg sodium carbonate, 5.0 kg sodium lauryl sulphate. In the second barrel segment is dosed 19.5 kg of a solution consisting of 17.5 kg water and 2 kg KOH/hour with a gear pump. In the 4^{th} segment is gravimetic dosed 230 kg naproxen/hour and the 4^{th} and 5^{th} segment of the barrel is kept at 80°C. Within the 6^{th} to 9^{th} segment of the barrel the mass is cooled down to 40°C. The totally solubilized mass is discharged in the form of a rope with a diameter of about 8mm. The material is in a solid, crystalline state and is immediately be milled through sieves with a mesh size of 5.0 mm and 2.0 mm. The dense granules have a particle size between 0.1-2.0 mm.

365,9 kg of this granulate is blended with 40 kg potassium hydrogen carbonate and 0.5 kg stearic acid for 15 minutes. The final blend is compressed to biconvex tablets with a diameter of 10.5 mm and a tablet weight of corresponding to 200 mg naproxen (406mg). The tablets have a perfectly smooth surface, a highly mechanical stability and a mean hardness of 72.5 N. The disintegration time at 37°C in water is 6.5-8.5 minutes.

### Example 21

In the first segment of a barrel of twin screw extruder are separately dosed per hour a mixture of 3.91 kg sodium hydroxide, containing 2.5% silicium dioxide and by a second gravimetric feeder a mixture of 8.0 kg glycine, 4.96 kg potassium chloride, 0.3 kg sodium lauryl sulphate/hour. In the 2^{nd} segment of the barrel is dosed by a gear pump a solution of 1.74 kg water and 0.49 kg potassium carbonate within 1 hour. The temperature in the 3^{rd} section is 60°C. Into the 4^{th} segment is dosed per hour 25.4kg ketoprofen. The temperature in the 4^{th} and 5^{th} segment of the barrel is 80°C. In the following 3 segments the temperature is decreased to 60 °C and the slit die is kept at 85°C. A white suspension is discharged through the die in the form of a tape with a dimension of 5cm/1mm. The tape solidifies on a conveyor belt within 5 seconds by cooling air of 25°C and can after 10 seconds be milled to a granulate with a particle size distribution of 0.1 - 2.0mm. The granulate was transferred into a fluid bed granulator and 100 kg granulate was sprayed with 20.5 kg of a 5% w/w aequeous solution of povidone K90. The temperature of the inlet air was 40°C. The obtained granulate was nearly dust free and had a water content of 8.8 w/w (loss on drying at 105°C within 30 minutes). Subsequently 100 kg granulate were mixed with 1.0 kg stearic acid. The final mixture was compressed into biconvex tablets having a tablet hardness of 80 - 110 N and a disintegration time in water, measured in accordance with the European Pharmacopoeia, of 5.1- 6.7 minutes. Tablet cores were exactly coated according to example 23.

### Example 22

In a rotating coating pan, 406 kg of the round, biconvex tablets obtained according to Example 20 and having a tablet weight of 406 mg (containing the equivalent amount of 200 mg Naproxen) are heated up to 45°C and then coated with 75 kg aqueous coating dispersion 1, containing:

| | |
|---|---|
| Opadry II85F Clear® (Colorcon Limited, Dartford Kent DA26QD, England) | 13.5 kg |
| Brown iron oxide | 0.6 kg |
| Titan dioxide | 0.9 kg |
| Water | 60 kg |
| | 75 kg |

with coating parameters of:

| | |
|---|---|
| inlet air temperature: | 70°C |
| product temperature | 35-45°C |

After about 2.5 hrs coating time, the film coated tablets are dried for 20 minutes under the same air conditions. The disintegration times of the film coated tablets, measured according to the European Pharmacopoeia, in water at 37°C are:

| | |
|---|---|
| film coated tablets: | 6.7-8.5 min |

Taken the film coated tablets in the mouth, the typical naproxen taste appears after 6-9 seconds.

**Coating dispersion 2:**

| | |
|---|---|
| Eudragit E PO (Degussa, Röhm GmbH D-64275 Darmstadt) | 0.8 kg |
| Sodium dodecyl sulphate | 0.08 kg |
| Stearic acid | 0.12 kg |
| Saccharine Sodium | 0.08 kg |
| Talc | 0.4 kg |
| Brown iron oxide | 0.06 kg |
| Water | 14.0 kg |
| | 15.5 kg |

The film coated tablets are sprayed under the same condition with coating dispersion 2 within 1 hour.

The disintegration time of the film coated tablets with coating dispersion 1 and 2 is hardly changed. The tablets disintegrate in water at 37°C within 8.0-10.0 minutes.

The time for appearance of the typical naproxen taste in the mouth is substantially prolonged to about 25-35 seconds. The sweetener can be in the coating dispersion 1 and/or coating dispersion 2. It is possible that the coating dispersion 2 is additionally flavoured.

Instead of coating dispersion 1 other well known typical coating dispersions could be used with polymers like methyl hydroxy propyl cellulose, hydroxy propyl cellulose, xanthan etc.

### Example 23

A granulator, fitted with a jacket (for heating and cooling), an impeller and chopper, is filled with 230 kg naproxen, 150 kg potassium carbonate, 5 kg sodium carbonate and 10kg sodium lauryl sulfate. After blending for 10 minutes, the powder blend is heated to a product temperature of 38°C. Under gentle stirring 3 kg isopropanol (0.8%) are added. After 70 minutes stirring, the thermosolubilisation is finished and 1 g granules dissolves easily within 10 seconds in 100 ml water at 37°C. The granules are dried under vacuum for 20 minutes.

The granules are sieved through 1.25 mm and then compressed with an external lubrication system (fluidised magnesium stearate) to biconvex tablet cores with a diameter of 10.5 mm and a tabet weight of 343 mg(containing 200mg naproxen).

Under a room condition of 20°C/25% rel. humidity the tablets can be easily compressed to a hardness of 90-110N. The disintegration time in water is 95-135 seconds. The tablet cores are coated according to example 23 with coating suspension 1 and 2. The disintegration time in water at 37°C is 3,7 minutes.
The naproxen release from film coated tablets obtained in examples 22, 23 was tested by the paddle method described in the current European Pharmacopoeia in the following two media:
- 1000ml 0,1 M hydrochloric acid (artificial gastric juice, pH 1.2)
- 1000ml USP buffer (pH 7.2), produced from 50 ml 0.2 M aqueous KH₂PO₄ solution and 34.7ml 0.2 M aqueous NaOH solution, and made up with water to 1000ml.

### Example 24

In the 1^{st} segment of an extruder barrel a mixture of 15kg potassium carbonate, 0.5kg sodium carbonate and 1.9kg saccharose palmitat is dosed per hour. In the 3^{rd} segment 24,2 kg fenoprofen is dosed per hour. Segment three and four are heated up to 75°C, in segment the mass is 6-9 cooled to 40°C. A plastic mass is discharged which can milled within seconds through a sieve with 2.0mm mesh size. Without any addition of water a highly water soluble naproxen granulate was formed.

The sieved granulate is blended with 1% magnesium stearate and a mass corresponding to 200mg naproxen is filled in HPMC-capsules, size 1. After disintegration of the capsule in water of 37°C the granulate dissolves within 35 seconds.

### Example 25

318 mg solubilized granulate (containing 200mg naproxen) according to example 20 is mixed with 50 mg potassium hydrogen carbonate, 116mg Isomalt, 3mg Aspartam, 10mg of grapefruit flavour and 3mg magnesium stearate. 500mg of this blend are filled in an alu-stickpack. The content of one stickpack dissolves without stirring in 100ml water of 20°C within 25 seconds. The solubilized naproxen particles become buoyant and dissolve. The matrix of the solubilised particles contain micro-CO₂ bubbles formed during the extrusion process.

## Claims

1. A process for producing a water-soluble form of a non-steroidal anti-inflammatory drug (NSAID) containing at least one carboxylic group and comprising at least two aromatic rings in its structure wherein at least a part of the NSAID is present in salt form, the process comprising the steps of: providing a mixture comprising solid NSAID and a first base which is selected from the group of bases which have a pH of at least 11 as 0.1 molar aqueous solution or dispersion, and reacting the NSAID and the first base in essentially dry state at a temperature of from 20°C to 90°C.

2. The process of claim 1, wherein the water-soluble form of the NSAID has the form of a granulate.

3. The process of claim 1 or 2, wherein the first base is selected from the group consisting of sodium hydroxide, potassium hydroxide, sodium carbonate, potassium carbonate, sodium glycinate, potassium glycinate and tribasic sodium and potassium phosphates and mixtures thereof.

4. The process of claim 3, wherein the sodium glycinate or potassium glycinate is prepared in-situ by reacting glycine with base.

5. The process of any one of the preceding claims, wherein the mixture comprises from 0.8 to 1.2 mole of the first base per mole of NSAID.

6. The process of any one of the preceding claims, wherein the mixture comprises at least 0.8 mole, preferably 0.9 to 1.5 mole of one or more basic compounds per mole of NSAID.

7. The process of any one of the preceding claims, wherein the mixture comprises less than 2.5 moles of water per mole of NSAID, preferably 0.1 to 2, more preferably 0.1 to 1.2 mole of water per mole of NSAID.

8. The process of any one of the preceding claims, wherein the NSAID is selected from the group consisting of diflunisal, flufenamic acid, mefenamic acid, niflumic acid, sulindac, indomethacin, tolmetin, diclofenac, flur-biprofen, fenbufen, fenoprofen, tiaprofenic acid, ketoprofen, acemetacin, and naproxen.

9. The process of any one of the preceding claims, wherein the mixture comprises two or more basic compounds.

10. The process of claim 9, wherein the first base is selected from the group consisting of sodium hydroxide, potassium carbonate, sodium glycinate and potassium glycinate and wherein the mixture comprises a second base which is selected from the group consisting of potassium hydroxide, sodium carbonate and tribasic sodium and potassium phosphates.

11. The process of any one of claims 9 or 10, wherein the mixture comprises at least one base having a pH of 7.5 to < 11 as 0.1 M aqueous solution or dispersion.

12. The process of claim 11, wherein the base is selected from the group consisting of trisodium citrate, tripotassium citrate, arginine, lysine, meglumine.

13. The process of any one of claims 9 to 12, wherein the mixture comprises at least one sodium-containing base and at least one potassium-containing base.

14. The process of claim 13, wherein the sodium-containing base(s) and the potassium-containing base(s) are present in a molar ratio of 1:20 to 20:1, preferably 1:9 to 9:1.

15. The process of any one of claims 13 or 14, wherein the mixture comprises sodium hydroxide in combination with potassium hydroxide or potassium carbonate; or potassium carbonate in combination with sodium carbonate or sodium hydroxide.

16. The process of any one of the preceding claims, wherein the mixture additionally comprises one or more pharmaceutically acceptable excipients.

17. The process of claim 16, wherein the one or more excipients are selected from the group consisting of fillers, binders, disintegrants, glidants and anti-precipitation agents.

18. The process of claim 16 or 17, wherein the mixture comprises one or more neutral and water-soluble excipients exhibiting a pH in water of about 7 and a solubility in water at 37°C of at least 5% (w/w), preferably at least 10% (w/w), most preferably at least 15%(w/w).

19. The process of any one of claims 16 to 18, wherein the mixture comprises up to 20 mole, preferably 0.25 to 4 mole, more preferably 0.5 to 1.5 mole of excipients per mole of NSAID.

20. The process of claim 18 or 19, wherein the one or more neutral and water-soluble excipients are selected from the group consisting of potassium chloride, mannitol, isomalt, polymeric compounds, non-crosslinked polyvinyl-pyrrolidiones, cellulose derivatives, microcrystalline cellulose, tensides, sodium laurylsulfate, saccharose palmitate, glycine and mixtures thereof.

21. The process of claim 20, wherein the mixture comprises glycine.

22. The process of claim 21, wherein the mixture comprises 0.2 to 1.5 mole, preferably 0.7 to 1.3 mole, more preferably 0.4 to 1 mole of glycine per mole of NSAID.

23. The process of any one of claims 20 to 22, wherein the mixture comprises potassium chloride.

24. The process of claim 23, wherein the mixture comprises 0.3 to 1 mole of potassium chloride per mole of NSAID.

25. The process of any one of the preceding claims, wherein the mixture comprises 1 to 15 %, preferably 1 to 9 %, more preferably 4 to 7% by weight of polymeric compounds, based on the total weight of the mixture.

26. The process of any one of the preceding claims, wherein the reaction is carried out in a mixing vessel.

27. The process of claim 26, wherein said mixing vessel comprises means for cooling and/or heating the mixture in said vessel.

28. The process of claim 26 or 27, wherein said mixing vessel is an extruder.

29. The process of claim 28, wherein the first base is selected from the group consisting of sodium hydroxide, potassium hydroxide, sodium glycinate, potassium glycinate, tribasic sodium phosphates, tribasic potassium phosphates and mixtures thereof.

30. The process of claim 29, wherein the first base comprises sodium hydroxide and/or potassium hydroxide.

31. The process of any one of claims 28 to 30, wherein the mixture comprises sodium hydroxide or a mixture of sodium hydroxide and potassium hydroxide.

32. The process of any one of the preceding claims, wherein the mixture additionally comprises a non-aqueous liquid selected from the group consisting of aliphatic C₁-C₄-alcohols, acetone and mixtures thereof.

33. The process of claim 32, wherein the mixture comprises isopropanol.

34. The process of claim 32 or 33, wherein first base is selected from the group consisting of sodium carbonate and potassium carbonate and mixtures thereof.

35. The process of claim 34, wherein the mixture comprises 0.8 to 1.2 mole of potassium carbonate and 0 to 0.4 mole of sodium carbonate per mole of NSAID.

36. The process of any one of claims 32 to 35, wherein the reaction is performed essentially in the absence of water.

37. The process of any one of claims 32 to 36, wherein the mixture comprises less than 0.25 moles of non-aqueous liquid per mole of NSAID.

38. The process of any one of claims 32 to 37, wherein the reaction is carried out at a temperature of from 40 to 60°C, preferably 50 to 60 °C.

39. A water-soluble form of NSAID containing at least one carboxylic group and comprising at least two aromatic rings in its structure wherein at least a part of the NSAID is present in salt form, obtainable according to the process of any one of the preceding claims.

40. The water-soluble form of an NSAID of claim 39 which is a granulate.

41. A pharmaceutical composition comprising the granulate of claim 40.

42. The pharmaceutical composition of claim 41, comprising additionally a basic compound selected from the group consisting of sodium and/or potassium hydrogencarbonate, sodium carbonate, potassium carbonate, tribasic sodium and potassium phosphates and mixtures thereof.

43. The pharmaceutical composition any one of claims 41 or 42, further comprising one or more pharmaceutically acceptable excipients.

44. The pharmaceutical composition of any one of claims 41 to 43, having the form of a tablet, film coated tablet, sugar coated tablet, granulate filled in sachets or stickpacks, or capsule.

## Patentansprüche

1. Verfahren zur Herstellung einer wasserlöslichen Form eines nichtsteroidalen Entzündungshemmers (NSAID), der mindestens eine Carboxylgruppe enthält und mindestens zwei aromatische Ringe in seiner Struktur aufweist, wobei mindestens ein Teil des NSAIDs in Salzform vorliegt und das Verfahren die Stufen umfasst, in denen eine Mischung bereitgestellt wird, die festes NSAID und eine erste Base umfasst, die ausgewählt ist aus der Gruppe von Basen mit einem pH-Wert von mindestens 11 als 0,1 molare wässrige Lösung oder Dispersion, und das NSAID und die erste Base in im Wesentlichen trockenem Zustand bei einer Temperatur von 20°C bis 90°C umgesetzt werden.

2. Verfahren nach Anspruch 1, bei dem die wasserlösliche Form des NSAIDs die Form eines Granulats hat.

3. Verfahren nach Anspruch 1 oder 2, bei dem die erste Base ausgewählt ist aus der Gruppe bestehend aus Natriumhydroxid, Kaliumhydroxid, Natriumcarbonat, Kaliumcarbonat, Natriumglycinat, Kaliumglycinat und Trinatrium- und -kaliumphosphaten sowie Mischungen davon.

4. Verfahren nach Anspruch 3, bei dem das Natriumglycinat oder Kaliumglycinat in situ durch Umsetzung von Glycin mit Base hergestellt wird.

5. Verfahren nach einem der vorhergehenden Ansprüche, bei dem die Mischung 0,8 bis 1,2 Mol der ersten Base pro Mol NSAID umfasst.

6. Verfahren nach einem der vorhergehenden Ansprüche, bei dem die Mischung mindestens 0,8 Mol, vorzugsweise 0,9 bis 1,5 Mol von einer oder mehreren basischen Verbindungen pro Mol NSAID umfasst.

7. Verfahren nach einem der vorhergehenden Ansprüche, bei dem die Mischung weniger als 2,5 Mol Wasser pro Mol NSAID, vorzugsweise 0,1 bis 2, insbesondere 0,1 bis 1,2 Mol Wasser pro Mol NSAID umfasst.

8. Verfahren nach einem der vorhergehenden Ansprüche, bei dem das NSAID ausgewählt ist aus der Gruppe bestehend aus Diflunisal, Flufenaminsäure, Mefenaminsäure, Nifluminsäure, Sulindac, Indomethacin, Tolmetin, Diclofenac, Flurbiprofen, Fenbufen, Fenoprofen, Tiaprofensäure, Ketoprofen, Acemetacin und Naproxen.

9. Verfahren nach einem der vorhergehenden Ansprüche, bei dem die Mischung zwei oder mehr basische Verbindungen umfasst.

10. Verfahren nach Anspruch 9, bei dem die erste Base ausgewählt ist aus der Gruppe bestehend aus Natriumhydroxid, Kaliumcarbonat, Natriumglycinat und Kaliumglycinat, und bei dem die Mischung eine zweite Base umfasst, die ausgewählt ist aus der Gruppe bestehend aus Kaliumhydroxid, Natriumcarbonat und Trinatrium- und -kaliumphosphaten.

11. Verfahren nach einem der Ansprüche 9 bis 10, bei dem die Mischung mindestens eine Base mit einem pH-Wert von 7,5 bis < 11 als 0,1 M wässrige Lösung oder Dispersion umfasst.

12. Verfahren nach Anspruch 11, bei dem die Base ausgewählt ist aus der Gruppe bestehend aus Trinatriumcitrat, Trikaliumcitrat, Arginin, Lysin, Meglumin.

13. Verfahren nach einem der Ansprüche 9 bis 12, bei dem die Mischung mindestens eine natriumhaltige Base und mindestens eine kaliumhaltige Base umfasst.

14. Verfahren nach Anspruch 13, bei dem die natriumhaltige(n) Base(n) und die kaliumhaltige(n) Base(n) in einem molaren Verhältnis von 1:20 bis 20:1, vorzugsweise 1:9 bis 9:1 vorliegen.

15. Verfahren nach einem der Ansprüche 13 oder 14, bei dem die Mischung Natriumhydroxid in Kombination mit Kaliumhydroxid oder Kaliumcarbonat umfasst, oder Kaliumcarbonat in Kombination mit Natriumcarbonat oder Natriumhydroxid umfasst.

16. Verfahren nach einem der vorhergehenden Ansprüche, bei dem die Mischung zusätzlich einen oder mehrere pharmazeutisch annehmbare Hilfsstoffe umfasst.

17. Verfahren nach Anspruch 16, bei dem der eine oder die mehreren Hilfsstoffe ausgewählt ist bzw. sind aus der Gruppe bestehend aus Füllstoffen, Bindemitteln, Sprengmitteln, Gleitmitteln und Antifällungsmitteln.

18. Verfahren nach Anspruch 16 oder 17, bei dem die Mischung einen oder mehrere neutrale und wasserlösliche Hilfsstoffe umfasst, die einen pH-Wert in Wasser von etwa 7 und eine Löslichkeit in Wasser bei 37°C von mindestens 5 % (Gew./Gew.), vorzugsweise mindestens 10 % (Gew./Gew.), am meisten bevorzugt mindestens 15 % (Gew./Gew.) besitzen.

19. Verfahren nach einem der Ansprüche 16 bis 18, bei dem die Mischung bis zu 20 Mol, vorzugsweise 0,25 bis 4 Mol, insbesondere 0,5 bis 1,5 Mol Hilfsstoffe pro Mol NSAID umfasst.

20. Verfahren nach Anspruch 18 oder 19, bei dem der eine oder die mehreren neutralen und wasserlöslichen Hilfsstoffe ausgewählt ist bzw. sind aus der Gruppe bestehend aus Kaliumchlorid, Mannitol, Isomalt, polymeren Verbindungen, nicht vernetzten Polyvinylpyrrolidonen, Cellulosederivaten, mikrokristalliner Cellulose, Tensiden, Natriumlaurylsulfat, Saccharosepalmitat, Glycin und Mischungen davon.

21. Verfahren nach Anspruch 20, bei dem die Mischung Glycin umfasst.

22. Verfahren nach Anspruch 21, bei dem die Mischung 0,2 bis 1,5 Mol, vorzugsweise 0,7 bis 1,3 Mol, insbesondere 0,4 bis 1 Mol Glycin pro Mol NSAID umfasst.

23. Verfahren nach einem der Ansprüche 20 bis 22, bei dem die Mischung Kaliumchlorid umfasst.

24. Verfahren nach Anspruch 23, bei dem die Mischung 0,3 bis 1 Mol Kaliumchlorid pro Mol NSAID umfasst.

25. Verfahren nach einem der vorhergehenden Ansprüche, bei dem die Mischung 1 bis 15 Gew.%, vorzugsweise 1 bis 9 Gew.%, insbesondere 4 bis 7 Gew.% polymere Verbindungen umfasst, bezogen auf das Gesamtgewicht der Mischung.

26. Verfahren nach einem der vorhergehenden Ansprüche, bei dem die Reaktion in einem Mischgefäß durchgeführt wird.

27. Verfahren nach Anspruch 26, bei dem das Mischgefäß Mittel zum Kühlen und/oder Erwärmen der Mischung in dem Gefäß umfasst.

28. Verfahren nach Anspruch 26 oder 27, bei dem das Mischgefäß ein Extruder ist.

29. Verfahren nach Anspruch 28, bei dem die erste Base ausgewählt ist aus der Gruppe bestehend aus Natriumhydroxid, Kaliumhydroxid, Natriumglycinat, Kaliumglycinat, Trinatriumphosphaten, Trikaliumphosphaten und Mischungen davon.

30. Verfahren nach Anspruch 29, bei dem die erste Base Natriumhydroxid und/oder Kaliumhydroxid umfasst.

31. Verfahren nach einem der Ansprüche 28 bis 30, bei dem die Mischung Natriumhydroxid oder eine Mischung aus Natriumhydroxid und Kaliumhydroxid umfasst.

32. Verfahren nach einem der vorhergehenden Ansprüche, bei dem die Mischung zusätzlich eine nichtwässrige Flüssigkeit ausgewählt aus der Gruppe bestehend aus aliphatischen C₁- bis C₄-Alkoholen, Aceton und Mischungen davon umfasst.

33. Verfahren nach Anspruch 32, bei dem die Mischung Isopropanol umfasst.

34. Verfahren nach Anspruch 32 oder 33, bei dem die erste Base ausgewählt ist aus der Gruppe bestehend aus Natriumcarbonat und Kaliumcarbonat und Mischungen davon.

35. Verfahren nach Anspruch 34, bei dem die Mischung 0,8 bis 1,2 Mol Kaliumcarbonat und 0 bis 0,4 Mol Natriumcarbonat pro Mol NSAID umfasst.

36. Verfahren nach einem der Ansprüche 32 bis 35, bei dem die Reaktion im Wesentlichen in Abwesenheit von Wasser durchgeführt wird.

37. Verfahren nach einem der Ansprüche 32 bis 36, bei dem die Mischung weniger als 0,25 Mol nichtwässrige Flüssigkeit pro Mol NSAID umfasst.

38. Verfahren nach einem der Ansprüche 32 bis 37, bei dem die Reaktion bei einer Temperatur von 40 bis 60°C, vorzugsweise 50 bis 60°C durchgeführt wird.

39. Wasserlösliche Form von NSAID, das mindestens eine Carboxylgruppe enthält und mindestens zwei aromatische Ringe in seiner Struktur aufweist, wobei mindestens ein Teil des NSAIDs in Salzform vorliegt, erhältlich nach dem Verfahren gemäß einem der vorhergehenden Ansprüche.

40. Wasserlösliche Form eines NSAIDs nach Anspruch 39, die ein Granulat ist.

41. Pharmazeutische Zusammensetzung, die das Granulat gemäß Anspruch 40 umfasst.

42. Pharmazeutische Zusammensetzung nach Anspruch 41, die zusätzlich eine basische Verbindung ausgewählt aus der Gruppe bestehend aus Natrium- und/oder Kaliumhydrogencarbonat, Natriumcarbonat, Kaliumcarbonat, Trinatrium- und -kaliumphosphaten und Mischungen davon umfasst.

43. Pharmazeutische Zusammensetzung nach einem der Ansprüche 41 oder 42, die ferner einen oder mehrere pharmazeutisch annehmbare Hilfsstoffe umfasst.

44. Pharmazeutische Zusammensetzung nach einem der Ansprüche 41 bis 43 in Form einer Tablette, einer filmbeschichteten Tablette, einer zuckerbeschichteten Tablette, in Sachets oder Stickpacks gefülltem Granulat, oder einer Kapsel.

## Revendications

1. Procédé pour la production d'une forme hydrosoluble d'un médicament anti-inflammatoire non stéroïdien (NSAID) contenant au moins un groupe carboxylique et comprenant au moins deux noyaux aromatiques dans sa structure, dans lequel au moins une partie du NSAID est présente sous forme de sel, le procédé comprenant les étapes consistant à : fournir un mélange comprenant un NSAID solide et une première base qui est choisie dans le groupe de bases qui ont un pH d'au moins 11 à l'état de solution ou dispersion aqueuse 0,1 molaire, et faire réagir le NSAID et la première base à l'état pratiquement anhydre à une température de 20°C à 90°C.

2. Procédé suivant la revendication 1, dans lequel la forme hydrosoluble du NSAID est sous forme de granules.

3. Procédé suivant la revendication 1 ou 2, dans lequel la première base est choisie dans le groupe consistant en l'hydroxyde de sodium, l'hydroxyde de potassium, le carbonate de sodium, le carbonate de potassium, le glycinate de sodium, le glycinate de potassium, le phosphate trisodique, le phosphate tripotassique et leurs mélanges.

4. Procédé suivant la revendication 3, dans lequel le glycinate de sodium ou le glycinate de potassium est préparé in situ par réaction de la glycine avec une base.

5. Procédé suivant l'une quelconque des revendications précédentes, dans lequel le mélange comprend 0,8 à 1,2 mol de la première base par mole de NSAID.

6. Procédé suivant l'une quelconque des revendications précédentes, dans lequel le mélange comprend au moins 0,8 mol, de préférence 0,9 à 1,5 mol, d'un ou plusieurs composés basiques par mole de NSAID.

7. Procédé suivant l'une quelconque des revendications précédentes, dans lequel le mélange comprend moins de 2,5 mol d'eau par mole de NSAID, avantageusement 0,1 à 2, plus avantageusement 0,1 à 1,2 mol d'eau par mole de NSAID.

8. Procédé suivant l'une quelconque des revendications précédentes, dans lequel le NSAID est choisi dans le groupe consistant en le diflunisal, l'acide flufénamique, l'acide méfénamique, l'acide niflumique, le sulindac, l'indométhacine, la tolmétine, le diclofénac, le flurbiprofène, le fenbufène, le fénoprofène, l'acide tiaprofénique, le kétoprofène, l'acémétacine et le naproxène.

9. Procédé suivant l'une quelconque des revendications précédentes, dans lequel le mélange comprend deux ou plus de deux composés basiques.

10. Procédé suivant la revendication 9, dans lequel la première base est choisie dans le groupe consistant en l'hydroxyde de sodium, le carbonate de potassium, le glycinate de sodium et le glycinate de potassium et dans lequel le mélange comprend une seconde base qui est choisie dans le groupe consistant en l'hydroxyde de potassium, le carbonate de sodium, le phosphate trisodique et le phosphate tripotassique.

11. Procédé suivant l'une quelconque des revendications 9 et 10, dans lequel le mélange comprend au moins une base ayant un pH de 7,5 ou moins de 11 sous forme d'une solution ou dispersion aqueuse 0,1 M.

12. Procédé suivant la revendication 11, dans lequel la base est choisie dans le groupe consistant en le citrate trisodique, le citrate tripotassique, l'arginine, la lysine et la méglumine.

13. Procédé suivant l'une quelconque des revendications 9 à 12, dans lequel le mélange comprend au moins une base contenant du sodium et au moins une base contenant du potassium.

14. Procédé suivant la revendication 13, dans lequel la ou les bases contenant du sodium et la ou les bases contenant du potassium sont présentes en un rapport molaire de 1:20 à 20:1, de préférence de 1:9 à 9:1.

15. Procédé suivant l'une quelconque des revendications 13 et 14, dans lequel le mélange comprend de l'hydroxyde de sodium en association avec de l'hydroxyde de potassium ou du carbonate de potassium ; ou bien du carbonate de potassium en association avec du carbonate de sodium ou de l'hydroxyde de sodium.

16. Procédé suivant l'une quelconque des revendications précédentes, dans lequel le mélange comprend en outre un ou plusieurs excipients pharmaceutiquement acceptables.

17. Procédé suivant la revendication 16, dans lequel le ou les excipients sont choisis dans le groupe consistant en des charges, des liants, des agents de délitement, des agents de glissement et des agents anti-précipitation.

18. Procédé suivant la revendication 16 ou 17, dans lequel le mélange comprend un ou plusieurs excipients neutres et hydrosolubles présentant un pH dans l'eau d'environ 7 et une solubilité dans l'eau à 37°C d'au moins 5 % (en poids/poids), avantageusement d'au moins 10 % (en poids/poids), de préférence d'au moins 15 % (en poids/poids).

19. Procédé suivant l'une quelconque des revendications 16 à 18, dans lequel le mélange comprend jusqu'à 20 mol, avantageusement 0,25 à 4 mol, plus avantageusement 0,5 à 1,5 mol d'excipients par mole de NSAID.

20. Procédé suivant la revendication 18 ou 19, dans lequel le ou les excipients neutres et hydrosolubles sont choisis dans le groupe consistant en le chlorure de potassium, le mannitol, l'isomalt, des composés polymères, des polyvinylpyrrolidones non réticulées, des dérivés de cellulose, la cellulose microcristalline, des agents tensioactifs, le laurylsulfate de sodium, le palmitate de saccharose, la glycine et leurs mélanges.

21. Procédé suivant la revendication 20, dans lequel le mélange comprend de la glycine.

22. Procédé suivant la revendication 21, dans lequel le mélange comprend 0,2 à 1,5 mol, avantageusement 0,7 à 1,3 mol, plus avantageusement 0,4 à 1 mol de glycine par mole de NSAID.

23. Procédé suivant l'une quelconque des revendications 20 à 22, dans lequel le mélange comprend du chlorure de potassium.

24. Procédé suivant la revendication 23, dans lequel le mélange comprend 0,3 à 1 mol de chlorure de potassium par mole de NSAID.

25. Procédé suivant l'une quelconque des revendications précédentes, dans lequel le mélange comprend 1 à 15 %, avantageusement 1 à 9 %, plus avantageusement 4 à 7 % en poids de composés polymères, sur la base du poids total du mélange.

26. Procédé suivant l'une quelconque des revendications précédentes, dans lequel la réaction est conduite dans un récipient de mélange.

27. Procédé suivant la revendication 26, dans lequel ledit récipient de mélange comprend des moyens pour le refroidissement et/le chauffage du mélange dans ledit récipient.

28. Procédé suivant la revendication 26 ou 27, dans lequel ledit récipient de mélange est une extrudeuse.

29. Procédé suivant la revendication 28, dans lequel la première base est choisie dans le groupe consistant en l'hydroxyde de sodium, l'hydroxyde de potassium, le glycinate de sodium, le glycinate de potassium, les phosphates trisodiques, les phosphates tripotassiques et leurs mélanges.

30. Procédé suivant la revendication 29, dans lequel la première base comprend de l'hydroxyde de sodium et/ou de l'hydroxyde de potassium.

31. Procédé suivant l'une quelconque des revendications 28 à 30, dans lequel le mélange comprend de l'hydroxyde de sodium ou un mélange d'hydroxyde de sodium et d'hydroxyde de potassium.

32. Procédé suivant l'une quelconque des revendications précédentes, dans lequel le mélange comprend en outre un liquide non aqueux choisi dans le groupe consistant en des alcools aliphatiques en C₁ à C₄, l'acétone et leurs mélanges.

33. Procédé suivant la revendication 32, dans lequel le mélange comprend de l'isopropanol.

34. Procédé suivant la revendication 32 ou 33, dans lequel la première base est choisie dans le groupe consistant en le carbonate de sodium, le carbonate de potassium et leurs mélanges.

35. Procédé suivant la revendication 34, dans lequel le mélange comprend 0,8 à 1,2 mol de carbonate de potassium et 0 à 0,4 mol de carbonate de sodium par mole de NSAID.

36. Procédé suivant l'une quelconque des revendications 32 à 35, dans lequel la réaction est conduite pratiquement en l'absence d'eau.

37. Procédé suivant l'une quelconque des revendications 32 à 36, dans lequel le mélange comprend moins de 0,25 mol de liquide non aqueux par mole de NSAID.

38. Procédé suivant l'une quelconque des revendications 32 à 37, dans lequel la réaction est conduite à une température de 40 à 60°C, de préférence de 50 à 60°C.

39. Forme hydrosoluble de NSAID contenant au moins un groupe carboxylique et comprenant au moins deux noyaux aromatiques dans sa structure, dans laquelle au moins une partie du NSAID est présente sous forme de sel, pouvant être obtenue suivant le procédé de l'une quelconque des revendications précédentes.

40. Forme hydrosoluble d'un NSAID suivant la revendication 39, qui est sous forme de granules.

41. Composition pharmaceutique comprenant les granules de la revendication 40.

42. Composition pharmaceutique suivant la revendication 40, comprenant en outre un composé basique choisi dans le groupe consistant en le carbonate acide de sodium et/ou de potassium, le carbonate de sodium, le carbonate de potassium, le phosphate trisodique, le phosphate tripotassique et leurs mélanges.

43. Composition pharmaceutique suivant l'une quelconque des revendications 41 et 42, comprenant en outre un ou plusieurs excipients pharmaceutiquement acceptables.

44. Composition pharmaceutique suivant l'une quelconque des revendications 41 à 43, sous forme d'un comprimé, d'un comprimé enrobé d'un film, d'un comprimé enrobé de sucre, de granules remplissant des sachets ou bâtonnets, ou d'une capsule.
